# EUROPÄISCHE PATENTSCHRIFT

(11) **EP 0 855 192 B1**
(45) Veröffentlichungstag und Bekanntmachung des Hinweises auf die Patenterteilung: **10.09.2003**
(21) Anmeldenummer: 97121095.0
(22) Anmeldetag: 01.12.1997
(51) Int. Cl.: A61M 1/36

(54) **Reperfusionsvorrichtung**
Reperfusion device
Dispositif de réperfusion

(30) Priorität: 24.01.1997 DE 19702402
(43) Veröffentlichungstag der Anmeldung: 29.07.1998
(73) Patentinhaber: Beyersdorf, Friedhelm, 79199 Kirchzarten (DE)
(72) Erfinder: Beyersdorf, Friedhelm, 79199 Kirchzarten (DE)
(74) Vertreter: Vetter, Ewald Otto, Dipl.-Ing.

(56) Entgegenhaltungen:
- EP-A- 0 210 424
- EP-A- 0 633 031
- DE-A- 4 003 425
- US-A- 4 259 952
- US-A- 4 336 802
- US-A- 5 322 500

## Beschreibung

Die Erfindung betrifft eine Reperfusionsvorrichtung.

Aus der DE 38 20 840 C1 ist eine wäßrige Reperfusionslösung zur Verminderung des Reperfusionsschadens nach akutem, peripheren Gefäßverschluß eines Patienten bekannt. Aus der EP 0 513 071 B1 ist eine Reperfusionsvorrichtung für die Reperfusion von Blutgefäßen bekannt, welche folgende Merkmale aufweist: Einen in ein Blutgefäß einführbarer Katheter, eine Blutentnahmevorrichtung zur Entnahme von oxigeniertem arteriellem Blut von einem Patienten, Mittel zum Mischen einer Reperfusionslösung mit dem vom Patienten entnommenen Blut, eine mechanische Pumpvorrichtung zur Förderung der Mischung aus Blut und Reperfusionslösung durch den Katheter in das Blutgefäß, und ein an der Katheterspitze angeordnetes Druckmeßmittel in Form eines Druckmeßlumens oder einer Drucksonde. Zur Beseitigung von Gefäßverschlüssen sind verschiedene Methoden bekannt. Trotz erfolgreicher Wiederherstellung der Blutstrombahn sind jedoch Morbidität und Mortalität nach einem solchen Gefäßeingriff relativ hoch. Die Hauptursache hierfür ist der Reperfusionsschaden, d.h. eine Gewebeschädigung die eintritt, wenn das Blut mit dem vom Herzen erzeugten vollen Arteriendruck nach Beseitigung des Gefäßverschlusses wieder durch die Arterie strömt. Dieser Nachteil wird durch Verwendung der genannten Reperfusionslösung verhindert oder wesentlich reduziert, wenn sie, beispielsweise mit der genannten Reperfusionsvorrichtung, nach Beseitigung des Gefäßverschlusses während einer längeren Zeitdauer von z.B. 30 Minuten und mit reduziertem Druck in das Gefäß eingebracht wird, bevor das Gefäß wieder mit dem vollen Arteriendruck belastet wird.

Durch die Erfindung soll die Aufgabe gelöst werden, eine Möglichkeit zu schaffen, durch welche eine Reperfusion mit Mitteln durchgeführt werden kann, welche kostengünstiger sind und einfacher gehandhabt werden können als die bekannten Verfahren und Vorrichtungen.

Diese Aufgabe wird gemäß der Erfindung durch die kennzeichnenden Merkmale des unabhängigen Ansprüchs 1 gelöst.

Weitere Merkmale der Erfindung sind in den Unteransprüchen enthalten.

Durch die Erfindung ist eine Vorrichtung, insbesondere ein Set für die Reperfusion, insbesondere für die Reperfusion am Bein eines Patienten gegeben, welches für die Gefäßchirurgie bezüglich kostengünstiger Herstellbarkeit und einfacher Handhabbarkeit akzeptabler ist als die bisher bekannten Mittel. Im Prinzip handelt es sich um die Verwendung von zwei Beuteln. Der erste Beutel wird mit dem Blut von der Becken-Arterie gefüllt. Dieses Blut wird dann in den zweiten Beutel befördert, in welchen zuvor eine bestimmte Menge einer Reperfusionsflüssigkeit gefüllt wurde. Anschließend wird diese Mischung aus Blut und Reperfusionsflüssigkeit während einer vorbestimmten Zeitdauer und mit einem vorbestimmten Druck in die Bein-Arterie geleitet. Dieses Verfahren wird mehrmals wiederholt. Alles läuft ohne eine Pumpe ab, weil jeder Beutel direkt mit der Hand oder indirekt durch eine von Hand betätigte Druckluftmanschette zusammengedrückt wird. Der Blutdruck wird in der Zufuhrleitung vom zweiten Beutel zum Bein oder in einer Zufuhrkanüle gemessen, mit welcher die Mischung in die Bein-Arterie eingeführt wird. Je nach der Druckgröße des Blutes in der Bein-Arterie wird der Druck in der Zufuhrleitung durch mehr oder weniger starken Druck auf den zweiten Beutel reguliert. Weil bei dieser Behandlung kein Heparin vorgesehen ist, müssen alle Beutel, Schläuche, Katheter und sonstigen Elemente, welche mit dem Blut oder dem Gemisch aus Blut und Reperfusionsflüssigkeit in Kontakt kommen, mit einer Heparinbeschichtung versehen werden.

Die Erfindung wird im folgenden mit Bezug auf die Zeichnungen anhand von bevorzugten Ausführungsformen beschrieben. Die Zeichnungen zeigen in
- Fig. 1: schematisch ein Set oder eine Vorrichtung zur Reperfusion gemäß der Erfindung,
- Fig. 2: schematisch eine weitere Ausführungsform der Erfindung.

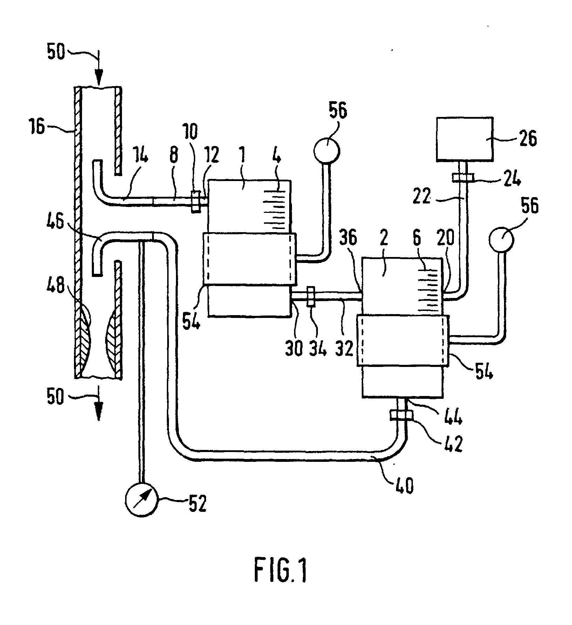

Das in Fig. 1 dargestellte Set zur Reperfusion von Blutgefäßen enthält zwei handelsübliche, von Hand zusammendrückbare flexible Beutel 1 und 2, die aus durchsichtigem oder mindestens licht-durchscheinendem, biegsamem Material bestehen und je mindestens eine Füllstand-Höhenmarkierung 4 oder 6 haben. Da die Beutel durchsichtig oder mindestens licht-durchscheinend sind, kann eine Bedienungsperson erkennen, ob der Füllstand einer Flüssigkeit in den Beuteln bereits eine gewünschte Höhe relativ zur Markierung 4 oder 6 erreicht hat.

Der erste Beutel 1 hat einen Blutentnahme-Schlauch 8, welcher mit einem Verschlußelement 10, vorzugsweise einer Schlauchklemme, wahlweise verschlossen oder geöffnet werden kann und einen Einlaß 12 des Beutels 1 im oberen Beutelbereich mit einem Blutentnahme-Katheter 14 verbindet. Der Blutentnahme-Katheter 14 wird in eine Arterie 16 des Patienten eingesetzt, damit durch den Blutdruck in der Arterie Blut in den ersten Beutel 1 bis zu der gewünschten Markierung 4 gefördert werden kann, wenn das Verschlußelement 10 geöffnet ist.

Ein Eingang 20 am oberen Endabschnitt des zweiten Beutels 2 ist über einen Reperfusionsflüssigkeits-Zufuhrschlauch 22, der durch ein Verschlußelement 24, vorzugsweise in Form einer Schlauchklemme, wahlweise geöffnet oder geschlossen werden kann, mit einem Behälter 26 verbunden, in welchem sich Reperfusionflüssigkeit befindet. Durch Öffnen des Verschlußelements 24 kann Reperfusionsflüssigkeit in den zweiten Beutel 2 gefüllt werden, bis die Reperfusionsflüssigkeit im zweiten Behälter 2 einen vorbestimmten Füllstand an seiner Höhenmarkierung 6 erreicht hat.

Ein Auslaß 30 am unteren Ende des ersten Beutels 1 ist durch einen Verbindungsschlauch 32, der durch ein Verschlußelement 34, vorzugsweise eine Schlauchklemme, wahlweise verschlossen oder geöffnet werden kann, mit einem zweiten Einlaß 36 am oberen Endabschnitt des zweiten Beutels 2 verbunden. Dadurch kann, wenn der Blutentnahmeschlauch 8 und der Reperfusionsflüssigkeits-Zufuhrschlauch 22 durch ihre Verschlußelemente 10 bzw. 24 geschlossen sind, eine dosierte Menge des Blutes aus dem ersten Behälter 1 in den zweiten Behälter 2 überführt und dort mit der zuvor dosierten Menge Reperfusionsflüssigkeit vermischt werden, wenn das Verschlußelement 34 des Verbindungsschlauches 32 geöffnet wird. Die dosierte Blutmenge ist vorzugsweise die Menge, die zuvor bis zu der vorbestimmten Höhenmarkierung 4 von der Arterie 16 in den ersten Beutel 1 gefüllt wurde.

Ein Reperfusions-Schlauch 40, welcher an seinem stromaufwärtigen Ende durch ein Verschlußelement 42, vorzugsweise eine Schlauchklemme, wahlweise geschlossen oder geöffnet werden kann, ist an seinem stromaufwärtigen Ende an einen Auslaß 44 am unteren Ende des zweiten Beutels 2 angeschlossen und hat an seinem stromabwärtigen Ende einen Reperfusions-Katheter 46, welcher in die gleiche Arterie 16 oder in eine andere Arterie des gleichen Patienten stromaufwärts von der Stelle einsetzbar ist, an welcher eine Gefäßverengung oder ein Gefäßverschluß 48 zuvor beseitigt wurde. Die arterielle Blut-Strömungsrichtung ist durch Pfeile 50 dargestellt. Dadurch kann durch Öffnen des Verschlußelementes 42 des Reperfusions-Schlauches 40, wenn zuvor die Verschlußelemente 24 und 34 des Reperfusionsflüssigkeits-Zufuhrschlauches 22 und des Verbindungsschlauches 32 geschlossen wurden, die zuvor dosierte Menge an Blut und Reperfusionsflüssigkeit aus dem zweiten Behälter 2 durch den Reperfusions-Katheter 46 in die Arterie gefördert werden, und zwar mit einem vorbestimmten Druck und während einer vorbestimmten Zeitdauer. Dieser Druck wird von einem Druckmeßgerät 52 optisch angezeigt, welches an den Reperfusionsflüssigkeits-Zufuhrschlauch 40 oder, vorzugsweise, direkt an den Reperfusions-Katheter 46 angeschlossen ist und den darin befindlichen Druck anzeigt. Die Zeitdauer, während welcher die Mischung aus Blut und Reperfusionsflüssigkeit in das Blutgefäß wie z.B. die Arterie 16 eingebracht wird, kann von einer Bedienungsperson an jeder beliebigen Uhr abgelesen werden und kann zum Beispiel ungefähr 30 Minuten betragen.

Zum Entleeren des Blutes aus dem ersten Behälter 1 in den zweiten Behälter 2 wird der erste Behälter 1 von einer Bedienungsperson zusammengedrückt. In gleicher Weise wird zum Entleeren des Blut-Reperfusionsflüssigkeits-Gemisches aus dem Behälter 2 in die Arterie 16 dieser Behälter 2 von einer Bedienungsperson zusammengedrückt. Das Zusammendrücken der beiden Beutel 1 und 2 kann von Hand entweder dadurch erfolgen, daß die Hand direkt auf die Beutel 1 oder 2 drückt, oder dadurch, daß die Beutel 1 und 2 in einer Quetschvorrichtung stehen oder von einer Quetschvorrichtung umgeben werden, vorzugsweise eine handelsübliche Druckmanschette, welche von Hand durch eine Bedienungsperson aufpumpbar ist. Fig. 1 zeigt eine Druckmanschette 54 mit einem von Hand bedienbaren Balg, Ballon oder Luftpumpe 56 zum wahlweise Aufpumpen oder Entlüften der Druckmanschette 54, um dadurch den ersten Beutel 1 zusammenzudrücken oder wieder zu entfalten. In gleicher Weise ist der zweite Beutel 2 vorzugsweise ebenfalls von einer handelsüblichen Druckmanschette 54 umgeben, welche einen von Hand bedienbaren Balg, Ballon oder Luftpumpe 56 aufweist, zum wahlweisen Aufpumpen oder Entlüften dieser Druckmanschette 54 und damit zum wahlweisen Zusammendrücken oder Entfalten des zweiten Beutels 2. Die Druckmanschetten 54 sind nur schematisch dargestellt und werden nicht im Detail beschrieben, da es sich um bekannte, handelsübliche Elemente handelt.

Die beiden Beutel 1, 2, alle Schläuche 8, 22, 32, 40, alle Schlauchklemmen 10, 34, 42, und die beiden Katheter 14, 46 sind handelsübliche Teile. Weil bei der Entfernung von Gefäßverschlüssen und bei der Reperfusion kein Heparin verwendet wird, müssen alle mit Blut in Kontakt kommenden Flächen, insbesondere die Innenflächen der Beutel 1 und 2 und der Leitungen 8, 14, 32 und 40, mit einer Schicht aus Heparin versehen sein.

Es kann folgendes Verfahren zum Mischen von Blut und einer Zusatzflüssigkeit für die Reperfusion von Blutgefäßen durchgeführt werden:
**1. Schritt**, Einbringen von Blut in den ersten Beutel 1 bis zu einer bestimmten Marke seiner Füllstands-Höhenmarkierung 4;
**2. Schritt**, unabhängig vom ersten Schritt, vor, gleichzeitig mit oder nach dem ersten Schritt, Fördern von Zusatzflüssigkeit aus dem Zusatzflüssigkeits-Behälter 26 in den zweiten Beutel 2 bis zu einer vorbestimmten Marke seiner Höhenmarkierung 6;
**3. Schritt**, Übertragen einer vorbestimmten dosierten Menge des Blutes aus dem ersten Beutel 1 in den zweiten Beutel 2, nachdem zuvor in den zweiten Beutel 2 eine vorbestimmte dosierte Menge der Zusatzflüssigkeit eingebracht wurde;
**4. Schritt**, Entleeren der Mischung aus Blut und Zusatzflüssigkeit oder einer dosierten Menge der Mischung aus dem zweiten Beutel.

Die beiden Beutel 1 und 2 können anstelle der Füllstand-Höhenmarkierungen 4 und/oder 6 andere Mittel, z.B. Schwimmer oder elektrische Elemente, aufweisen, welche die Füllstandshöhe der Beutel 1 und 2 anzeigen. In diesem Falle brauchen die Beutel 1 und 2 gegebenenfalls nicht aus durchsichtigem oder lichtdurchlässigem Material zu bestehen. Die Füllstands-Anzeigemittel dienen dazu, eine dosierte Menge des Blutes mit einer dosierten Menge der Reperfusionsflüssigkeit zu mischen und eine dosierte Menge einer solchen Mischung durch den Reperfusionskatheter 46 abzugeben.

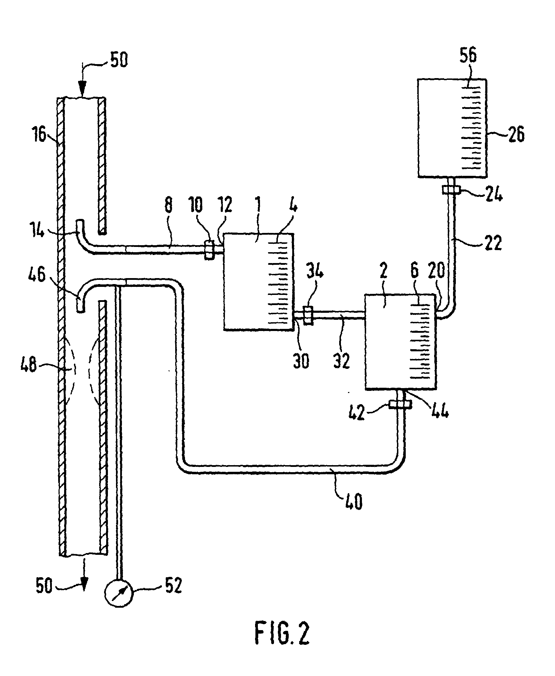

Es können Beutel 1 und 2 verwendet werden, welche keine Füllstands-Anzeigemittel (4, 6 oder andere) haben, wenn die dosierten Mengen anders definierbar sind. Beispielsweise zeigt Fig. 2 eine bevorzugte Ausführungsform, bei welcher der Behälter 26 Füllstands-Anzeigemittel 56 aufweist. Daran kann optisch die dosierte Menge Reperfusionsflüssigkeit abgelesen werden, die aus dem Behälter 26 in den zweiten Beutel 2 übertragen wird. In diesem Fall braucht der zweite Beutel 2 keine Füllstands-Anzeigemittel 6 zu haben.

Der Behälter 26 kann aus durchsichtigem oder lichtdurchlässigem Material bestehen und seine Füllstands-Anzeigemittel 56 können an ihm vorgesehene Markierungen sein.

Gemäß einer nochmals anderen bevorzugten Ausführungsform braucht der erste Beutel 1 keine Füllstands-Anzeigemittel, z.B. 4, zu haben, wenn mindestens der zweite Beutel 2, oder dieser zweite Beutel 2 und der Behälter 26 welche (6, 56) haben. In diesem Fall kann zunächst vom ersten Beutel 1 soviel Blut in den zweiten Beutel 2 übertragen werden durch den Schlauch 32, bis im zweiten Beutel 2 eine bestimmte Füllstandshöhe erreicht ist; dann wird aus dem Behälter 26 soviel Reperfusionsflüssigkeit in den zweiten Beutel 2 übertragen, bis im zweiten Beutel 2 eine bestimmte zweite Füllstandshöhe erreicht ist oder bis die Füllstandshöhe im Behälter 26 auf einen bestimmten Wert abgefallen ist.

Da es schwierig ist, den zweiten Beutel 2 vollständig zu entleeren, ist seine Füllstandsanzeige 6 kein zuverlässiges Maß für genau dosierte Mengen. Deshalb werden vorzugsweise das Blut durch Füllstands-Anzeigemittel 4 des ersten Beutels 1 dosiert und die Reperfusionsflüssigkeit durch Füllstands-Anzeigemittel 56 des Behälters 26 dosiert.

An den zweiten Beutel 2 können weitere Beutel der gleichen Art angeschlossen sein, um dem Blut weitere Flüssigkeiten in dosierten Mengen beizumischen.

Anstatt der bevorzugten Schlauchklemmen 10, 24, 34 und 42 können Hähne, Ventile oder andere Verschlußmittel verwendet werden.

## Patentansprüche

1. Reperfusionsvorrichtung für die Reperfusion von Blutgefäßen eines Patienten, mit Mitteln zur Entnahme von Blut von dem Patienten, zum Mischen des entnommenen Blutes mit einer Reperfusionslösung in einem vorbestimmten Mischungsverhältnis, und zum Fördern der Mischung in das betreffende Blutgefäß,
**dadurch gekennzeichnet, daß** mindestens zwei Beutel (1, 2) aus biegsamem Material vorgesehen sind, welche zum Entleeren zusammendrückbar sind und dann vor einem erneuten Füllen wieder aufrichtbar sind;
daß ein erster (1) dieser Beutel (1, 2) mit einer Blutentnahme-Katheterleitung (8, 14) zur Blutentnahme von dem Patienten versehen ist;
daß die Beutel (1, 2) je durch einen Schlauch miteinander verbunden sind, so daß eine vorbestimmte Menge Blut des ersten Beutels (1) in den letzten Beutel (2) übertragbar ist;
daß an einen der Beutel (2), mit Ausnahme des ersten Beutels (1), über einen Schlauch ein Vorratsbehälter (26) für Reperfusionsflüssigkeit angeschlossen ist, so daß eine vorbestimmte Menge Reperfusionsflüssigkeit von dem Vorratsbehälter (26) in diesen Beutel (2) übertragbar ist, wobei dieser Beutel (2), falls er nicht der letzte Beutel (2) ist, mit dem letzten Beutel (2) über einen Schlauch (32) strömungsmäßig verbunden ist zur Übertragung dieser dosierten Menge Reperfusionsflüssigkeit;
daß der letzte Beutel (2) mit einer Reperfusions-Katheterleitung (40, 46) versehen ist, um aus ihm eine dosierte Menge eines Gemisches bestehend aus der dosierten Menge des Blutes und der dosierten Menge der Reperfusionsflüssigkeit abzugeben;
daß Mittel (4, 6, 56) zur optischen Anzeige der dosierten Menge Blut und der dosierten Menge Reperfusionsflüssigkeit vorgesehen sind;
daß Verschlußelemente (10, 24, 34, 42) zum wahlweisen Öffnen oder Schließen der Strömungswege vorgesehen sind, welche durch die Schläuche (22, 32) und Katheter-Leitungen (8, 14, 40, 46) gebildet sind.

2. Reperfusionsvorrichtung nach Anspruch 1,
**dadurch gekennzeichnet, daß** mindestens einer der Beutel (1, 2) aus einem durchsichtigen oder lichtdurchscheinendem Material besteht, so daß seine Füllstandshöhe von außen optisch erkennbar ist.

3. Reperfusionsvorrichtung nach Anspruch 2,
**dadurch gekennzeichnet, daß** der oder die durchsichtigen oder lichtdurchscheinenden Beutel (1, 2) mit einer Füllstands-Höhenmarkierung (4, 6) versehen ist oder versehen sind.

4. Reperfusionsvorrichtung nach einem der vorhergehenden Ansprüche,
**dadurch gekennzeichnet, daß** mindestens einer der Beutel (1, 2) aus Kunststoff besteht.

5. Reperfusionsvorrichtung nach einem der vorhergehenden Ansprüche,
**dadurch gekennzeichnet, daß** der Vorratsbehälter (26) Füllstands-Anzeigemittel (56) aufweist.

6. Reperfusionsflüssigkeit nach Anspruch 5,
**dadurch gekennzeichnet, daß** der Vorratsbehälter (26) aus durchsichtigem oder lichtdurchscheinendem Material besteht, und daß die Füllstands-Anzeigemittel eine Füllstands-Höhenmarkierung (56) am Vorratsbehälter (26) aufweisen.

7. Reperfusionsvorrichtung nach einem der vorhergehenden Ansprüche,
**dadurch gekennzeichnet, daß** mindestens einer der Beutel (1, 2) mit einer von Hand aufblasbaren und anschließend wieder entlüftbaren Druckmanschette (54) versehen ist, um ihn wahlweise zusammenzudrücken und dann wieder zu entfalten.

8. Reperfusionsvorrichtung nach einem der vorhergehenden Ansprüche,
**dadurch gekennzeichnet, daß** mindestens eines der Verschlußmittel (10, 24, 34, 42) eine Schlauchklemme ist.

9. Reperfusionsvorrichtung nach einem der vorhergehenden Ansprüche,
**dadurch gekennzeichnet, daß** alle mit Blut in Kontakt kommenden Flächen, insbesondere die Innenflächen der Beutel (1, 2) und der Leitungen (8, 14, 32, 40, 46), mit einer Schicht aus Heparin versehen sind.

10. Reperfusionsvorrichtung nach einem der vorhergehenden Ansprüche,
**dadurch gekennzeichnet, daß** die Reperfusions-Katheterleitung (40, 46) mit einem Druckmeßgerät (52) zur optischen Anzeige des in ihr herrschenden Druckes versehen ist.

## Claims

1. Reperfusion device for the reperfusion of blood vessels of a patient, using means for removing blood from the patient, for mixing the removed blood with a reperfusion solution in a predetermined mixing ratio, and for conveying the mixture into the relevant blood vessel, **characterised in that** at least two pouches (1, 2) made from flexible material are provided, which can be pressed together for emptying and can then be erected again before renewed filling; **in that** a first (1) of these pouches (1, 2) is provided with a blood-removal catheter line (8, 14) for blood removal from the patient; **in that** the pouches (1, 2) are connected to one another in each case by a hose, so that a predetermined quantity of blood of the first pouch (1) can be transferred into the last pouch (2); **in that** a supply container (26) for reperfusion liquid is connected to one of the pouches (2), with the exception of the first pouch (1), via a hose, so that a predetermined quantity of reperfusion liquid can be transferred from the supply container (26) to this pouch (2), wherein this pouch (2), if it is not the last pouch (2), is connected according to flow to the last pouch (2) via a hose (32) to transfer this metered quantity of reperfusion liquid; **in that** the last pouch (2) is provided with a reperfusion catheter line (40, 46) in order to release from it a metered quantity of a mixture consisting of the metered quantity of blood and the metered quantity of reperfusion liquid; **in that** means (4, 6, 56) for visual display of the metered quantity of blood and the metered quantity of reperfusion liquid are provided; **in that** closure elements (10, 24, 34, 42) are provided for alternate opening or closing ofthe flow paths which are formed by the hoses (22, 32) and catheter lines (8, 14, 40, 46).

2. Reperfusion device according to claim 1, **characterised in that** at least one of the pouches (1, 2) consists of a transparent or light-transparent material, so that its filling level can be seen visually from the outside.

3. Reperfusion device according to claim 2, **characterised in that** the transparent or light-transparent pouch or pouches (1, 2) is provided or are provided with a filling level height marking (4, 6).

4. Reperfusion device according to one of the preceding claims, **characterised in that** at least one of the pouches (1, 2) consists of plastic.

5. Reperfusion device according to one of the preceding claims, **characterised in that** the supply container (26) has filling level display means (56).

6. Reperfusion liquid according to claim 5, **characterised in that** the supply container (26) consists of transparent or light-transparent material, and **in that** the filling level display means have a filling level height marking (56) on the supply container (26).

7. Reperfusion device according to one of the preceding claims, **characterised in that** at least one of the pouches (1, 2) is provided with a pressure cuff (54) which can be inflated by hand and then can be relieved again in order alternately to press it together and then to unfold it again.

8. Reperfusion device according to one of the preceding claims, **characterised in that** at least one of the closure means (10, 24, 34, 42) is a hose clip.

9. Reperfusion device according to one of the preceding claims, **characterised in that** all surfaces coming into contact with blood, in particular the inner surfaces of the pouches (1, 2) and the lines (8, 14, 32, 40, 46), are provided with a layer of heparin.

10. Reperfusion device according to one ofthe preceding claims, **characterised in that** the reperfusion catheter line (40, 46) is provided with a pressure-measuring device (52) for the visual display of the pressure prevailing in it.

## Revendications

1. Dispositif de reperfusion pour la reperfusion de vaisseaux sanguins d'un patient, avec des moyens de prélèvement de sang du patient, afin de mélanger le sang prélevé avec une solution de reperfusion dans un rapport de mélange prédéterminé et afin de faire avancer le mélange dans le vaisseau sanguin concerné ;
**caractérisé en ce qu'**au moins deux poches (1, 2) en matériau flexible sont prévues, lesquelles sont comprimables pour se vider et reprennent ensuite leur forme initiale avant de renouveler le remplissage ;
**en ce qu'**une première (1) de ces poches (1, 2) est pourvue d'un cathéter de prélèvement de sang (8, 14) pour prélever le sang d'un patient ;
**en ce que** les poches (1, 2) sont toutes reliées entre elles par un tuyau flexible, de sorte qu'une quantité prédéterminés de sang de la première poche (1) est transférable dans la dernière poche (2) ;
**en ce qu'**une des poches (2), à l'exception de la première poche (1), est reliée à un réservoir (26) pour liquide de reperfusion par un tuyau, de sorte qu'une quantité prédéterminée de liquide de reperfusion du réservoir (26) est transférable dans cette poche (2), moyennant quoi cette poche (2), si elle n'est pas la dernière poche (2), est reliée selon le flux à la dernière poche (2) par un tuyau (32) pour transfuser cette quantité dosée de liquide de reperfusion ;
**en ce que** la dernière poche (2) est pourvue d'un cathéter de reperfusion (40, 46), pour délivrer à partir de lui une quantité dosée d'un mélange composé à partir de la quantité dosée de sang et de la quantité dosée de liquide de reperfusion ;
**en ce que** des moyens (4, 6, 56) d'affichage optique de la quantité dosée de sang et de la quantité dosée de liquide de reperfusion sont prévus ;
**en ce que** des éléments obturateurs (10, 24, 34, 42) sont prévus pour ouvrir ou fermer facultativement les voies de flux, lesquelles sont formées des tuyaux flexibles (22, 32) et de conduits de cathéters (8, 14, 40, 46).

2. Dispositif de reperfusion selon la revendication 1,
**caractérisé en ce qu'**au moins une des poches (1, 2) se compose d'un matériau transparent ou translucide, de sorte que son niveau de remplissage soit visible de l'extérieur.

3. Dispositif de reperfusion selon la revendication 2,
**caractérisé en ce que** la ou les poche(s) transparents(s) ou translucide(s) (1, 2) est (sont) pourvue(s) d'un marquage de niveau de remplissage (4, 6).

4. Dispositif de reperfusion selon l'une quelconque des revendications précédentes,
**caractérisé en ce qu'**au moins une des poches (1, 2) se compose de matière plastique.

5. Dispositif de reperfusion selon l'une quelconque des revendications précédentes,
**caractérisé en ce que** le réservoir (26) comprend un indicateur de niveau de remplissage (56).

6. Dispositif de reperfusion selon la revendication 5,
**caractérisé en ce que** le réservoir (26) est composé d'un matériau transparent ou translucide et que les indicateurs de niveau de remplissage comprennent un marquage de niveau de remplissage (56) sur le réservoir (26).

7. Dispositif de reperfusion selon l'une quelconque des revendications précédentes,
**caractérisé en ce qu'**au moins une des poches (1, 2) est pourvue d'une manchette à pression gonflable à la main et ensuite à nouveau ventilable (54), afin éventuellement de le comprimer et ensuite de le déployer de nouveau.

8. Dispositif de reperfusion selon l'une quelconque des revendications précédentes,
**caractérisé en ce qu'**au moins un des obturateurs (10, 24, 34, 42) est une pince pour tuyau souple.

9. Dispositif de reperfusion selon l'une quelconque des revendications précédentes,
**caractérisé en ce que** toutes les surfaces venant en contact avec le sang, en particulier les surfaces intérieures des poches (1, 2) et des conduits (8, 14, 32, 40, 46), sont pourvues d'une couche d'héparine.

10. Dispositif de reperfusion selon l'une quelconque des revendications précédentes,
**caractérisé en ce que** le conduit de cathéter de reperfusion (40, 46) est pourvu d'un instrument de mesure (52) pour un affichage optique de la pression qui y règne.
